# EUROPEAN PATENT SPECIFICATION

(11) **EP 2 120 816 B1**
(45) Date of publication and mention of the grant of the patent: **23.05.2012**
(21) Application number: 08710120.0
(22) Date of filing: 21.02.2008
(51) Int. Cl.: A61F 6/12, A61F 13/28

(54) **PESSARY AND LUBRICANT SYSTEM AND METHOD**
PESSAR SOWIE SCHMIERMITTELSYSTEM UND -VERFAHREN DAFÜR
PESSAIRE ET SYSTÈME ET PROCÉDÉ LUBRIFIANT

(30) Priority: 21.02.2007 US 902413 P
(43) Date of publication of application: 25.11.2009
(73) Proprietor: The Procter & Gamble Company, Cincinnati, OH 45202 (US)
(72) Inventor: CARACCI, David, Joseph, Cincinnati, Ohio 45241 (US); HARTSELL, Debra, Lynn, Cincinnati, Ohio 45208 (US); MACAULAY, Frank, Delmar, Cincinnati, Ohio 45255 (US); MASON, Michael, Wayne, Cincinnati, Ohio 45249 (US)
(74) Representative: Kremer, Véronique Marie Joséphine
(86) International application number: PCT/IB2008/050632
(87) International publication number: WO 2008/102315

(56) References cited:
- WO-A-99/11210
- US-A- 4 421 504
- US-A- 4 620 534
- US-A1- 2002 026 140
- US-A1- 2004 078 013

## Description

### FIELD OF INVENTION

This application relates to pessary devices for relief of female incontinence. More particularly, the present invention relates to pessary devices having a lotion coating on a surface thereof, and to systems for application of a lotion coating to either or both of the pessary device or the body.

### BACKGROUND OF THE INVENTION

Urinary incontinence, in which the ordinary bodily muscle functions fail to prevent unintended leakage of urine, is a common malady among women, particularly older women. It is estimated that up to 50% of women occasionally leak urine involuntarily, and that approximately 25% of women will seek medical advice at some point in order to deal with the problem. Stress incontinence, the most common type of urinary incontinence, refers to the involuntary loss of urine resulting from abdominal pressure rise, occurring during exercise, coughing, sneezing, laughing, etc. When stress incontinence occurs, it is usually the result of the abnormal descent of the urethra and bladder neck below the level of the pelvic floor. Many women wear sanitary napkins or diapers in order to deal with incontinence, and some women resort to surgical procedures.

Pessary devices are known to help relieve involuntary urination in a female. Such devices can be elastic and designed for arrangement in the vagina for compressive action on and support of the neck of the bladder. In some instances, pessary devices can elastically expand or unfold such that a portion presses against the anterior wall of the vagina and another portion presses against the posterior wall of the vagina, thereby bearing against the urethra.

One drawback to the use of pessary devices is the lack of comfort during insertion or withdrawal. Typically delivered using applicators similar to those used for tampons, pessary devices introduce discomfort during use due to their relatively larger size.

Another drawback to the use of pessary devices is the lack of natural lubrication in the vagina during use. Because pessaries can be used during non-menstrual periods, and for postmenopausal uses, dryness and friction during insertion and removal can be a problem.

Even for tampon uses, first time users and use during times of light loading can result in insertion and removal discomfort.

Lubricated applicators for placing an insert in a body cavity are disclosed in US 4,421,504, US 2004/0078013 A1 and US 2002/0026140 A1. An apparatus for insertion of an intravaginal article is described in US 4,620,534. A lubricant dispenser is disclosed in WO 99/11210.

It would be beneficial to have a treatment for pessary devices that can enable reduce discomfort during insertion.

Additionally, it would be beneficial to have a treatment method and device for reducing discomfort during withdrawal of a pessary device.

Further, it would be beneficial to have a method and system for easing discomfort due to insertion or withdrawal, with the method and system being easy and discreet to use, transport, and dispose.

### SUMMARY OF THE INVENTION

A device comprising a tubular applicator and lotion dispenser in combination is disclosed. The device includes a tubular applicator and a lotion dispenser for applying lotion to the tubular applicator. The lotion dispenser includes a lotion selected from the group consisting of gels, solutions, compositions, lubricants, and creams.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1 is an exploded perspective view of a first embodiment of an pessary applicator according to the present invention.
FIG. 2 is a perspective view of an embodiment of an pessary for use with the applicators of the present invention.
FIG. 3 is a perspective view of an embodiment of an pessary for use with the applicators of the present invention.
FIG. 4 is a front view of the an embodiment of a pessary applicator, housing the pessary of FIG. 2.
FIG. 5 is a perspective view of an embodiment of the pessary applicator in the pessary housing position, and also shows the pessary member of FIG. 2 in phantom.
FIG. 6 is a perspective view of an embodiment of the pessary applicator in the pessary discharging position, and also shows the pessary member of FIG. 2 discharged from the pessary applicator.
FIG. 7 is a front view of an embodiment of the pessary applicator, housing the pessary of FIG. 2.
FIG. 8 is a perspective view of a pessary applicator and a lotion dispenser, showing lotion on both the dispenser and on the insertion end of the applicator.
FIGS. 9-12 show various embodiments of lotion dispensers.

### DETAILED DESCRIPTION OF THE INVENTION

The present invention is directed to pessary devices, or pessaries, which are inserted into the female vagina and expandable such that pressure is exerted on the urethra to help control involuntary urinary incontinence. In general, pessaries are delivered into the vagina in a similar manner as are tampons, by use of a plastic or cardboard tubular applicator comprising an outer member and in inner member, the inner member sometimes described as a plunger or an actuator, and used to push the pessary out of an open end of the outer member. In general, the improvement of the invention can be enjoyed for tampons and tampon applicators. Therefore the description below of a pessary device is not to be limiting to the full scope of the invention, as indicated by the claims herein.

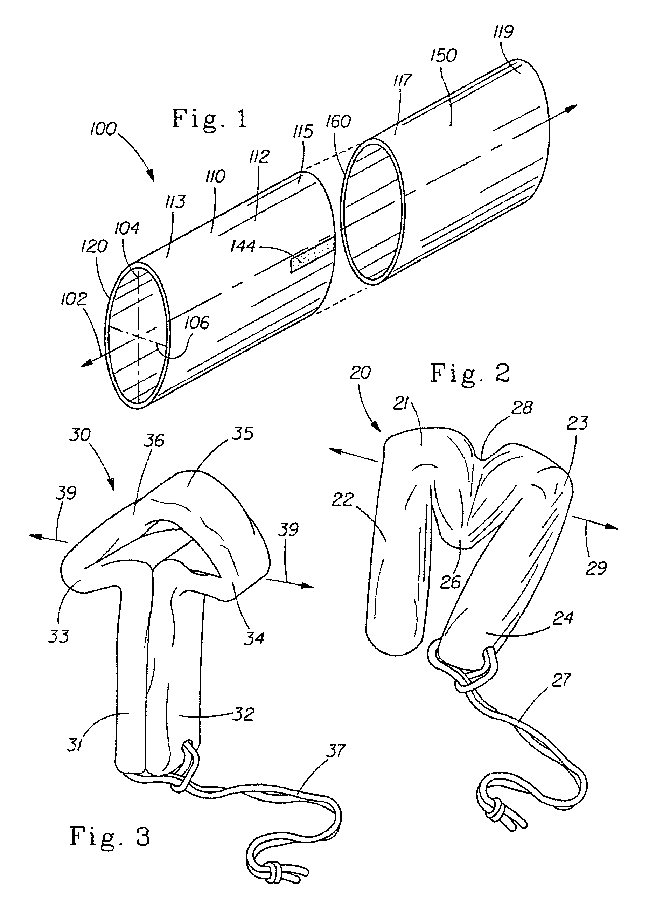

Referring now to the drawings and initially to FIG. 1, a pessary applicator is shown generally at **100** in a pre-assembled configuration. The applicator **100** has an outer member **110** defined by an pessary carriage or pessary housing, and an inner member **150** that can be referred to as a plunger or actuator. The outer member **110** can be in the shape of a hollow cylinder **112** extending the entire longitudinal length of the outer member **110**, that is, from a leading, vaginal insertion end portion **113** to a trailing end portion **115**. The cylinder **112** can extend substantially parallel to a longitudinal axis **102** of the applicator **100**. In a direction substantially perpendicular to its longitudinal axis **102**, the outer member **110** can be circular, or can be a non-circular, substantially elliptical or oval cross-section having a major axis **104** defined by the longest diameter of the cross-section, and a minor axis **106** defined by the shortest diameter of the cross-section. The outer member can have finger grips, flanges, grooves or indentions as is known in the art of tampon applicators. The outer member **110** houses a pessary (not shown) that is to be discharged into the vaginal cavity.

The inner member **150** can share the common longitudinal axis **102** with the outer member **110**, and extends from an insertion end portion **117** to a trailing end portion **119**. The inner member **150** is sized and adapted to be moveable within the outer member **110,** and is operable to discharge the pessary from a pessary discharging exit end **120** of the outer member **110**. The pessary discharging exit end **120** is the leading end of the applicator **100** when the applicator is inserted into the vagina. Preferably, as shown, the inner member **150** has a similar cross-section as the outer member, and can also have a substantially oval or elliptical cross-section in a direction substantially perpendicular to its longitudinal axis **102**. The cross-section of the outer member **110** can be oval or elliptical (or substantially elliptical) rather than circular so as to more accurately approximate the shape of the vaginal opening, thereby facilitating the insertion of the applicator **100** into the vagina without discomfort to the user, while at the same time providing a volume that maximizes the space for housing a pessary member.

The applicator **100** can also include one or more orientation indicators. In the embodiment shown in FIG. 1, the orientation indicator is in the form of a marker **144**, and is located on the outer surface of the outer member **110**. The marker **144** is positioned so as to indicate the relative rotational position of the applicator **100**, and hence, allows the user to correctly orient the pessary inside the vagina. In the embodiment shown, the marker **144** is located on the minor axis of the elliptical cross-section of the outer member **110**.

FIGS. 2 and 3 illustrate exemplary pessary devices. A pessary device, or more particularly an "incontinence pessary device" as used herein refers to devices specifically designed, configured, and/or adapted for placement into a vagina in order to reduce the occurrence and/or severity of female urinary incontinence. While pessary devices are typically made of non-absorbent materials, at least partially absorbent materials or absorbent materials may also be used. Examples of pessary devices that can be employed in the present invention are shown in WO 2005/087154 published 22 September 2005; WO 2004/103213, published 2 December 2004; US 6,090,038 granted 18 July 2000; US 5,618,256 granted 8 April 1997; and US 6,739,340 granted 25 May 2004.

In FIG. 2, a pessary member having a "M-shape" profile in a plane parallel to the directions indicated by arrows **29**, is shown generally at **20**. Pessary **20** can be formed from an elongated member having a first end portion **22** and a second end portion **24**, and has three folds, **21**, **23**, and **26**. The first end portion **22** and the second end portion **24** are aligned generally adjacent each other and, together with the folds **21**, **23**, **26**, form a generally planar M-shaped profile. An inward crease **28** is formed in a direction generally perpendicular to the plane of the M-shaped profile. One of the end portions is attached to a withdrawal member, such as a string **27**. The pessary member **20** can include, or can be made from, a resilient material that functions to expand the pessary **20** in at least a direction indicated by arrows **29** once the pessary **20** is placed within the vagina.

In order for the pessary **20** to be most effective in reducing the occurrence and/or severity of incontinence, the pessary **20** should be properly placed and correctly oriented within the vagina. Specifically, the pessary **20** should expand in the direction **29** so as to press against and/or compress the urethra and support the urinary sphincter muscle, thereby reducing the occurrence and/or severity of incontinence.

FIG. 3 illustrates another embodiment of a pessary useful for reducing the occurrence and/or severity of incontinence. Briefly, the pessary **30** can be formed from an elongated member that includes a first end portion **31** and a second end portion **32**, and a connecting portion **36**. The first end portion **31** and the second end portion **32** are aligned adjacent each other, and the connecting portion 36 includes three folds **33**, **34**, **35** in order to form a generally planar closed loop dome-shaped profile. The dome-shaped profile is in a plane parallel to the direction indicated by arrows **39**. In this embodiment, both end portions can be attached to a withdrawal member, such as a string **37**. The pessary **30** can include, or is can be made from, a resilient material that functions to expand the pessary **30** in at least a direction indicated by arrows **39** once the pessary **30** is placed within the vagina. Like the pessary of FIG. 2, the pessary **30** should be properly placed and oriented within the vagina to maximize its effectiveness. The pessary **30** can expand in the direction **39** so as to press against and/or compress the urethra and support the urinary sphincter muscle, thereby reducing the occurrence and/or severity of incontinence.

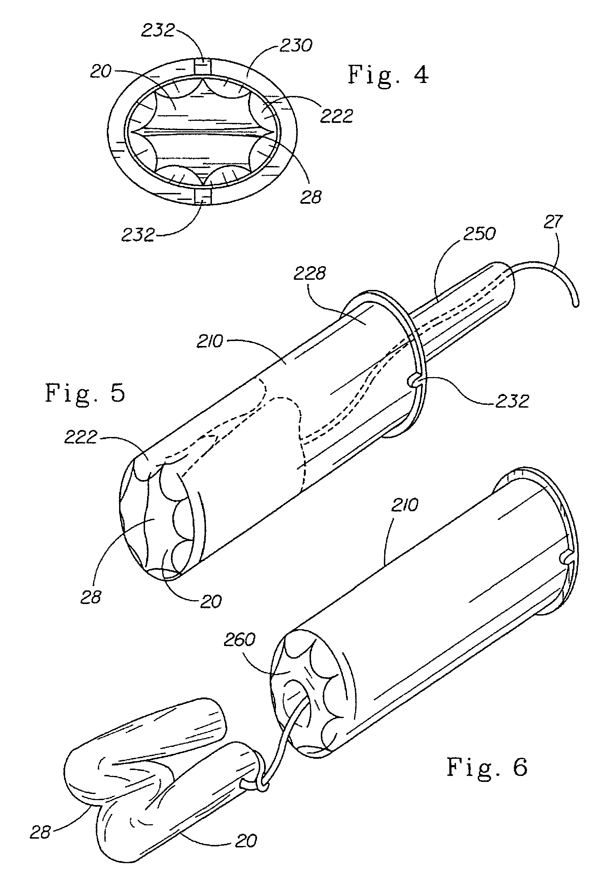

FIGS. 4 to 6 illustrate an applicator **200** with the pessary **20** of FIG. 2. FIG. 4 illustrates a front cross-sectional view of the applicator **200**, shown housing the pessary **20**. FIG. 5 illustrates the pessary **20** in phantom within the outer member **210**, and inner member **250**. FIG. 6 illustrates the pessary discharged from the outer member **210** after inner member **250** has been pushed into outer member **210**. As shown, the inward crease **28** of the pessary **20** is substantially parallel to the major axis of the elliptical cross-section, and substantially perpendicular to the minor axis of the elliptical cross-section and an imaginary line connecting the orientation indicators **232**. The pessary **20** is therefore oriented within the outer member **210** such that the plane defined by the M-shaped profile of the pessary **20** is substantially parallel to the minor axis of the substantially elliptical cross-section.

Those skilled in the art will recognize that applicator shapes and sizes and pessary shapes and sizes can be varied as desired to meet the incontinence needs of the user. Those skilled in the art will appreciate that various modifications may be made to the pessary devices shown. For example, the applicator may be round in cross section, and the pessary may be an un-folded, expandable device. Likewise, the manners of attachment of the withdrawing member shown in FIGS. 2 and 3 are only exemplary, and other manners known in the art can be used equally as well. Likewise, those skilled in the art will appreciate that various modifications may be made to applicators **100** and **200**, such as modifications known in the art of tampon insertion.

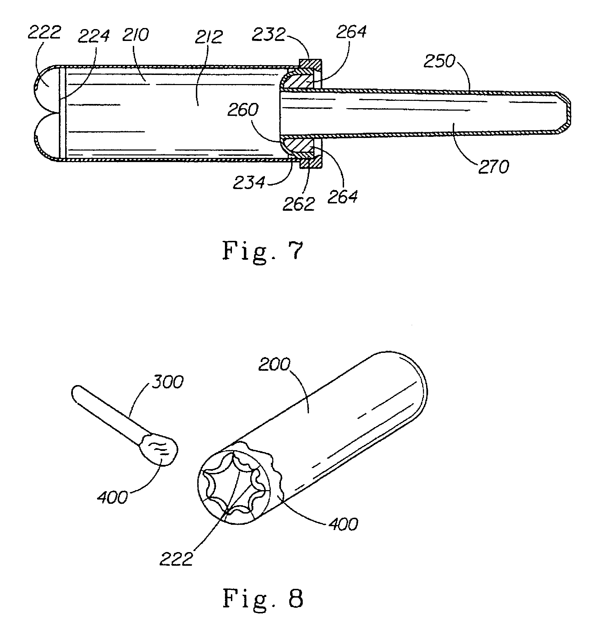

FIG. 7 illustrates the applicator **200** in a partial cutaway view of an assembled configuration. The inner member **250** of the applicator **200** includes a head portion **260**, a shank portion **270** and a trailing portion **275**. The head portion **260** rests in the trailing end **240** of the outer member **210** with the extensions **262** slideably engaging the inner surface of the outer member **210**. The head portion **260** can be held in place longitudinally by a retaining rib **234**. The applicator **200** can be formed of a material, such as a thermoplastic, that allows for slight deformation of the extensions **262** when the inner member **250** is pushed by the user, thereby allowing the extensions **262** to pass the retaining rib **234**, and to allow the inner member **250** to advance through the interior of the outer member **210**.

The head portion **260** can be integrally formed with or joined to the shank portion **270** by a continuous return flange **265** extending from the exit **290** back towards the trailing portion **275**. The flange **265** is preferably reinforced by support members **264** connected with the outer surface of the shank portion **270**. The flange **265** includes a plurality of outer member engaging portions defined by extensions **262** for contacting the interior of the outer member **210**. Although the flange **265** shown extends a relatively short length relative to the full length of the inner member **250**, in other embodiments, the flange **265** extends significantly longer relative to the length of the inner member **250**.

The shank portion **270** can have a substantially smaller cross-section than the head portion **260**. Because of this smaller cross-section, the user is able to actuate the inner member **250** with a single finger of the user's same hand that is holding the outer member **210** at the gripping portion **228**. Furthermore, the smaller cross-section shank portion **270** is more aesthetically pleasing because it makes the applicator as a whole appear smaller and less likely to cause discomfort in its use.

The above description of a pessary and pessary applicator is provided as exemplary of devices benefiting by the improvement of the present invention. For a more full description of the exemplary pessary applicator, see US 6,645,136, issued November 11, 2003 to Zunker et al.

In one embodiment pessary 20 can be inserted by a non-tubular applicator. Pessary 20 can be inserted by pushing into the vagina with a rigid insertion member, such as a wooden stick, plastic stick, or other suitable member. Such pessaries and insertion devices are disclosed in US 5,618,256, granted 8 April 1997. Therefore, in one embodiment, an applicator can be a device that does not surround the pessary prior to use, but is an applicator that acts to push the pessary in, much like the inner member of the above-described pessary applicators **100** or **200**.

The present invention is an improvement in the art of pessary devices by providing in combination with a pessary insertion device such as applicator **200**, a lotion application device, and a method and system for application of a lotion to either the applicator **100** or **200**, or the body of the user. As shown in FIG. 8, lotion **400** can be dispensed from a lotion dispenser **300** onto the insertion end tip of applicator **200**, including the petals **222**. Lotion **400** on the tip of an applicator **200** can aid in minimizing or eliminating the discomfort associated with insertion of the applicator into the vagina for proper insertion of the pessary into the vagina. Although useful for tampon applicators, the benefit can be enjoyed more for pessary applicators because pessaries are intended to be placed higher in the vagina, such that the applicator may need to be inserted farther into the vagina than a woman is comfortable with in the un-lotioned condition.

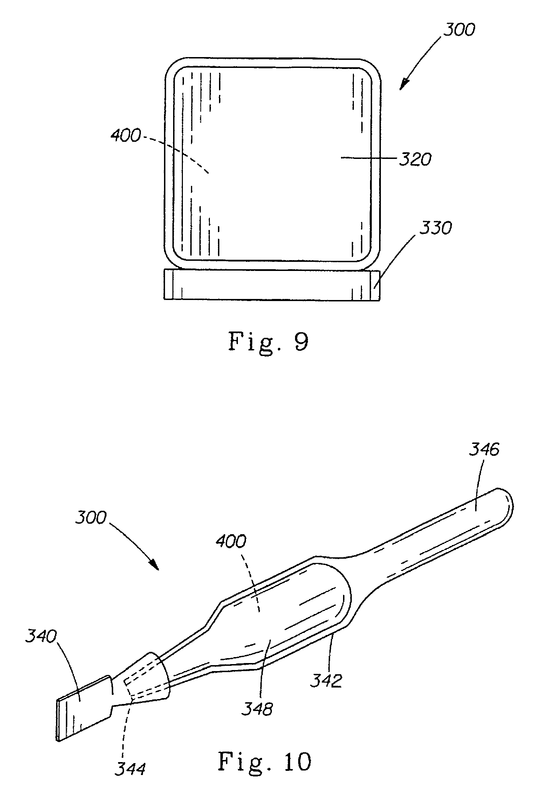

As shown in FIG. 9, a lotion dispenser **300** in the form of an enclosed pouch can be utilized to dispense lotion on the tip of applicator **200**, such as onto the flanges defining petals **222**, which can soften the vaginal insertion process. Lotion dispenser **300** can dispense a lotion **400**, which can be any of gels, solutions, compositions, lubricants, creams, or other lotions known to ease the discomfort caused by the introduction of an object into the vagina. Exemplary lotions include those intended to reduce frictional discomfort, including those of the type marketed by or similar to K-Y® brand jelly and lubricants. Other lubricants include solid microspheres, mineral oil, and Surgilube® chlorhexidine gluconate.

Lotion dispenser **300** can be any of known devices utilized to dispense viscous fluids. For example, lotion dispenser **300** can be of the type currently marketed by The Procter and Gamble Co. as Olay® brand eye derma-pod. Such as device is shown in FIG. 9, and can include a flexible foil pouch **320**, sealed about the edges and containing therein a suitable lotion or cream for vaginal use. Upon squeezing a sufficient between the finger and thumb with a sufficient force, the pouch **320** breaks and lotion or cream is released into a soft, resilient spongy tip **330**, which can be a foam sponge applicator. In use, the user can squeeze lotion, cream, gel, or other lubricant into soft, resilient spongy tip **330** as necessary to rub or wipe onto applicator **200** or onto the body portion to be contacted by applicator **200**. Pouch **320** can serve as a handle to provide a finger hold portion such that the user can apply lotion to either the tip of an applicator, to her body, or both prior to, or after insertion of pessary device. The size, length, and overall dimensions of applicator **300** shown in FIG. 9 can be tailored for use as necessary. In one embodiment, for example, the dimension of soft, resilient spongy tip **330** can be sized such that it extends beyond the largest dimension of pouch **320** so that discomfort due to the pouch pushing on any body parts is minimized or eliminated. In one embodiment, the overall size is approximately 2.5 cm on a side and can be folded to be generally flattened for storage and shipping. The pouch **320** and spongy tip **330** can be adhered together by means known in the art, and the entire dispenser can be separately packaged in a separate wrapper or box.

Lotion dispenser **300** can be of the type currently marketed by Alcon® as Tears Naturale Free®, which is a generally rigid, plastic device depicted in FIG. 10. As shown, dispenser **300** can have a removable and reusable cap **340** that can be frangibly joined to body **342** which can be integral with handle **346**. Between the capped tip and the handle is a reservoir portion **348** from which lotion, cream, gel, or other lubricant **400** can be squeezed out. In use, the user can twist off cap **340** and squeeze lotion or lubricant **400** from open tip **344**. Handle **346** provides a finger hold portion such that the user can use the dispenser to apply lotion to either the tip of an applicator, to her body, or both prior to or after insertion of pessary device. The size, length, and overall dimensions of applicator **300** shown in FIG. 10 can be tailored for use as necessary. In one embodiment, the overall length is about 8-10 cm and the handle portion is about 2-3 cm.

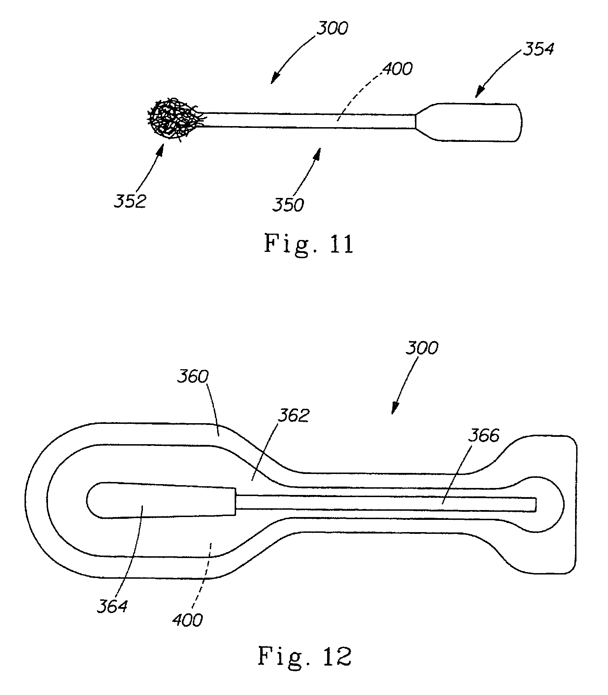

Lotion dispenser **300** can be of the type currently marketed as Baby Orajel® teething swabs, as depicted in FIG. 11. As shown, dispenser **300** can have a hollow round tube **350** that is frangibly connected and openable to a cotton-swabbed tip **352**. In use, the user can squeeze the other end **354** of dispenser **300** to cause pressure to break a frangible seal between lotion, cream, gel, or other lubricant **400** inside tube **350** and cotton-swabbed tip **352**, thereby permitting lotion to be absorbed into or forced into cotton-swabbed tip **352**. Hollow tube **350** provides a finger hold portion such that the user can apply lotion to either the tip of an applicator, to her body, or both prior to, or after insertion of pessary device. The size, length, and overall dimensions of applicator **300** shown in FIG. 11 can be tailored for use as necessary. In one embodiment, the overall length of tube **350** can be about 8-10 cm, and the diameter of a tube having a round cross section can be from about 1 to about 5 mm. Tube materials can include any plastic materials as known in the art. In one embodiment, tube **350** can be transparent, such that lotion **400** quantity can be observed.

Lotion dispenser **300** can be of the type currently marketed as Zicam® No-Drip Liquid Nasal Gel, as depicted in plan view in FIG. 12. As shown, dispenser **300** can have a foil backing layer **360** to which a clear plastic bubble packaged element **362** is bonded and which encloses a Q-tip®-like swab **364**. A lotion, cream, gel, or other lubricant **400** is also enclosed with swab **364**. In use, the user can open the dispenser **300** by tearing the plastic bubble element away from the foil backing and removing swab **364**. The "stick" portion **366** of swab **364** provides a finger hold portion such that the user can apply lotion to either the tip of an applicator, to her body, or both prior to, or after insertion of pessary device. The size, length, and overall dimensions of applicator **300** shown in FIG. 12 can be tailored for use as necessary. In one embodiment, the foil backing **360** can have a length of about 4-8 cm, and a width of about 3-3 cm. The swab **364** can have dimensions typical of swabs, including Q-tip® swabs.

Lotion dispenser **300** can be of the type currently marketed as 3M Cavilon® No Sting Barrier Film, which is a swab type plastic dispenser.

In one embodiment, lotion dispenser **300** can include commonly available items such as a simple swab, such as a Q-tip® swab marketed by Johnson Diversey. Lotion dispenser can also incorporate cotton balls, sponges, wipes, or items useful for spreading fluid or viscous fluids onto a surface.

The method of the present invention includes providing an applicator, such as a tampon applicator or pessary applicator. Applicator can be a plastic or cardboard tubular applicator, such as applicators **100** and **200** shown herein. The method also includes providing a lotion, cream, or other suitable lubricant **400** that can be utilized to lessen the discomfort associated with insertion or removal of a pessary, including the frictional discomfort caused by the applicator during insertion, and the general discomfort caused by removal of an expanded pessary after use. For insertion, the user can dispense by squeezing, wiping, or otherwise placing lotion on or near the tip or insertion end of the applicator, and can also put lotion on any other portion of the applicator or vaginal area thought necessary for increasing comfort of insertion. For withdrawal, the user can squeeze, wipe, or otherwise place lotion in, on or near the vaginal opening, and can also put lotion on any other portion of the body thought necessary for increasing comfort of withdrawal of the pessary device.

Lotion dispensers **300** can be packaged with pessary devices **20** or packaged separately. If packaged with pessary devices, dispensers **300** can be joined to the pessary device, such as by being adhered to the packaging of the device, or dispensers **300** can be enclosed in the packaging of the pessary device. In another embodiment, dispensers can be separately packaged, such as in a separate openable and sealable container. In one embodiment pessary devices can be individually packaged, and an individually packaged lotion dispenser can be associated each individually packaged pessary device. In one embodiment, individually packaged lotion dispensers can be packaged loosely with individually wrapped pessary devices. In one embodiment individually wrapped lotion dispensers can be adhered by adhesive, tape, or other joining means, to the packaging of individually wrapped pessary devices.

Lotion dispensers can be packaged in reusable, openable carrying cases, such as soft or rigid plastic cases that can be carried in a purse or handbag.

The above disclosure applies to tampons and tampon applicators as well. For either tampon applicators or pessary applicators, starter kits for new users can include combinations of applicators and lotion dispensers. In some embodiments, two lotion dispensers can be packaged with each applicator, whereby a first lotion can first be dispensed onto applicator, and later a second lotion, which may be different than the first lotion, can be dispensed onto the vaginal opening for removal of the tampon or pessary device.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from scope of the invention. It is therefore intended to cover in the appended claims all such changes and modifications that are within the scope of this invention.

## Claims

1. A device comprising a tubular applicator (100, 200) and lotion dispenser (300) in combination, the device comprising:
a. said tubular applicator (100, 200); and
b. said lotion dispenser (300) for applying lotion (400) to said tubular applicator (100, 200), said lotion dispenser (300) comprising a lotion (400) selected from the group consisting of gels, solutions, compositions, lubricants, and creams,
**characterized in that** said lotion dispenser (300) further comprises a swab.

2. The device of Claim 1, wherein said applicator (100, 200) is a pessary device and encloses a pessary.

3. The device of any one of the preceding claims, wherein said applicator (100, 200) is generally rigid and in the shape of a stick.

4. The device of any one of the preceding claims, wherein said lotion dispenser (300) is a first lotion dispenser, and the device further comprises a second lotion dispenser; and wherein said first lotion dispenser comprises a first lotion, and said second lotion dispenser comprises a second lotion that is different from said first lotion.

5. The device of any one of Claims 1 or 3-4, wherein said applicator (100, 200) is a tampon device and encloses a tampon.

6. The device of any one of the preceding claims, wherein said lotion dispenser (300) comprises frangible openings made to be opened by the application of finger pressure.

7. The device of any one of the preceding claims, wherein said lotion dispenser (300) comprises a hollow tube comprising said lotion (400), said hollow tube having at one end said swab.

8. The device of any one of the preceding claims, wherein said lotion dispenser (300) further comprises a foil pouch.

9. A method for preparing a pessary device for inserting a pessary into the vagina, said method comprising:
a. providing an applicator (100, 200) having an insertion end;
b. providing a lotion dispenser (300) comprising a swab and a lotion (400); and
c. dispensing said lotion (400) on said insertion end of said applicator (100, 200).

10. The method of Claim 9, wherein said lotion dispenser (300) comprises frangible openings made to be opened by the application of finger pressure.

11. The method of any one of Claims 9-10, wherein said lotion dispenser (300) comprises a hollow tube comprising said lotion (400), said hollow tube having at one end said swab.

12. The method of any one of Claims 9-11, wherein said lotion dispenser (300) further comprises a foil pouch.

## Patentansprüche

1. Vorrichtung, umfassend einen röhrenförmigen Applikator (100, 200) und einen Lotionspender (300) in Kombination, wobei die Vorrichtung Folgendes umfasst:
a. den röhrenförmigen Applikator (100, 200) und
b. den Lotionspender (300) zum Auftragen von Lotion (400) auf den röhrenförmigen Applikator (100, 200), wobei der Lotionspender (300) eine Lotion (400) umfasst, ausgewählt aus der Gruppe bestehend aus Gelen, Lösungen, Zusammensetzungen, Gleitmitteln und Cremes, **dadurch gekennzeichnet, dass** der Lotionspender (300) ferner einen Tupfer umfasst.

2. Vorrichtung nach Anspruch 1, wobei der Applikator (100, 200) eine Pessarvorrichtung ist und ein Pessar einschließt.

3. Vorrichtung nach einem der vorstehenden Ansprüche, wobei der Applikator (100, 200) im Allgemeinen starr und stabförmig ist.

4. Vorrichtung nach einem der vorstehenden Ansprüche, wobei der Lotionspender (300) ein erster Lotionspender ist und die Vorrichtung ferner einen zweiten Lotionspender umfasst, und wobei der erste Lotionspender eine erste Lotion umfasst und der zweite Lotionspender eine zweite Lotion umfasst, die sich von der ersten Lotion unterscheidet.

5. Vorrichtung nach einem der Ansprüche 1 oder 3-4, wobei der Applikator (100, 200) eine Tamponvorrichtung ist und ein Tampon einschließt.

6. Vorrichtung nach einem der vorstehenden Ansprüche, wobei der Lotionspender (300) reißbare Öffnungen umfasst, die so hergestellt sind, dass sie durch Ausübung von Fingerdruck geöffnet werden können.

7. Vorrichtung nach einem der vorstehenden Ansprüche, wobei der Lotionspender (300) eine hohle Röhre umfasst, welche die Lotion (400) umfasst, wobei die hohle Röhre an einem Ende den Tupfer aufweist.

8. Vorrichtung nach einem der vorstehenden Ansprüche, wobei der Lotionspender (300) ferner einen Folienbeutel umfasst.

9. Verfahren zum Herstellen einer Pessarvorrichtung zum Einführen eines Pessars in die Vagina, wobei das Verfahren Folgendes umfasst:
a. Bereitstellen eines Applikators (100, 200), der ein Einführende aufweist,
b. Bereitstellen eines Lotionspenders (300), umfassend einen Tupfer und eine Lotion (400), und
c. Abgeben der Lotion (400) auf das Einführende des Applikators (100, 200).

10. Verfahren nach Anspruch 9, wobei der Lotionspender (300) reißbare Öffnungen umfasst, die so hergestellt sind, dass sie durch Ausübung von Fingerdruck geöffnet werden können.

11. Verfahren nach einem der Ansprüche 9-10, wobei der Lotionspender (300) eine hohle Röhre umfasst, welche die Lotion (400) umfasst, wobei die hohle Röhre an einem Ende den Tupfer aufweist.

12. Verfahren nach einem der Ansprüche 9-11, wobei der Lotionspender (300) ferner einen Folienbeutel umfasst.

## Revendications

1. Dispositif comprenant un applicateur tubulaire (100, 200) et un distributeur de lotion (300) en combinaison, le dispositif comprenant :
a. ledit applicateur tubulaire (100, 200) ; et
b. ledit distributeur de lotion (300) pour appliquer une lotion (400) audit applicateur tubulaire (100, 200), ledit distributeur de lotion (300) comprenant une lotion (400) choisie dans le groupe constitué de gels, solutions, compositions, lubrifiants, et crèmes, **caractérisé en ce que** ledit distributeur de lotion (300) comprend en outre un tampon.

2. Dispositif selon la revendication 1, dans lequel ledit applicateur (100, 200) est un dispositif de type pessaire et entoure un pessaire.

3. Dispositif selon l'une quelconque des revendications précédentes, dans lequel ledit applicateur (100, 200) est généralement rigide et sous la forme d'un bâtonnet.

4. Dispositif selon l'une quelconque des revendications précédentes, dans lequel ledit distributeur de lotion (300) est un premier distributeur de lotion, et le dispositif comprend un deuxième distributeur de lotion ; et dans lequel ledit premier distributeur de lotion comprend une première lotion, et ledit deuxième distributeur de lotion comprend une deuxième lotion qui est différente de ladite première lotion.

5. Dispositif selon l'une quelconque des revendications 1 ou 3 à 4, dans lequel ledit applicateur (100, 200) est un dispositif à tampon et enferme un tampon.

6. Dispositif selon l'une quelconque des revendications précédentes, dans lequel ledit distributeur de lotion (300) comprend des ouvertures frangibles destinées à être ouvertes par l'application d'une pression du doigt.

7. Dispositif selon l'une quelconque des revendications précédentes, dans lequel ledit distributeur de lotion (300) comprend un tuyau creux comprenant ladite lotion (400), ledit tuyau creux ayant à une extrémité ledit tampon.

8. Dispositif selon l'une quelconque des revendications précédentes, dans lequel ledit distributeur de lotion (300) comprend en outre une pochette en pellicule.

9. Procédé de préparation d'un dispositif de type pessaire permettant d'insérer un pessaire dans le vagin, ledit procédé comprenant :
a. la fourniture d'un applicateur (100, 200) ayant une extrémité d'insertion ;
b. la fourniture d'un distributeur de lotion (300) comprenant un tampon et une lotion (400) ; et
c. la distribution de ladite lotion (400) sur ladite extrémité d'insertion dudit applicateur (100, 200).

10. Procédé selon la revendication 9, dans lequel ledit distributeur de lotion (300) comprend des ouvertures frangibles destinées à être ouvertes par l'application d'une pression du doigt.

11. Procédé selon l'une quelconque des revendications 9 à 10, dans lequel ledit distributeur de lotion (300) comprend un tuyau creux comprenant ladite lotion (400), ledit tuyau creux ayant à une extrémité ledit tampon.

12. Dispositif selon l'une quelconque des revendications 9 à 11, dans lequel ledit distributeur de lotion (300) comprend en outre une pochette en pellicule.
